# FASCICULE DE BREVET EUROPEEN

(11) **EP 3 977 966 B1**
(45) Date de publication et mention de la délivrance du brevet: **17.05.2023**
(21) Numéro de dépôt: 21199295.3
(22) Date de dépôt: 28.09.2021
(51) Int. Cl.: A61F 5/01

(54) **GENOUILLÈRE CONFIGURÉE POUR COOPÉRER AVEC LES MALLÉOLES ET/OU LE COU-DE-PIED**
KNIEORTHESE, DIE SO GESTALTET IST, DASS SIE MIT DEN KNÖCHELN UND/ODER DEM RIST ZUSAMMENWIRKT
KNEE PAD CONFIGURED TO ENGAGE WITH THE ANKLE BONES AND/OR THE INSTEP

(30) Priorité: 30.09.2020 FR 2009952
(43) Date de publication de la demande: 06.04.2022
(73) Titulaire: Lesage, Alexandre, 35000 Rennes (FR); Lesage, Patrick, 35350 Saint Meloir des Ondes (FR); Ricordeau, Laurent, 44100 Nantes (FR)
(72) Inventeur: Lesage, Alexandre, 35000 Rennes (FR); Lesage, Patrick, 35350 Saint Meloir des Ondes (FR); Ricordeau, Laurent, 44100 Nantes (FR)
(74) Mandataire: Cabinet Beau de Loménie

(56) Documents cités:
- WO-A1-2014/191895
- WO-A1-2015/106317
- BE-A- 901 688
- DE-A1-102006 011 465
- US-A- 4 494 534
- US-A1- 2020 197 209
- US-B2- 6 524 265
- US-B2- 10 682 249

## Description

### Domaine Technique

Le présent exposé concerne une genouillère configurée pour coopérer avec les malléoles et/ou le cou-de-pied.

### Technique antérieure

Les genouillères sont des dispositifs médicaux configurés pour restreindre tout ou partie des mouvements de l'articulation du genou d'un membre inférieur. Ce type de dispositif est couramment utilisé pour des pathologies médicales variées, et il en existe de nombreux types configurés pour traiter ces différentes pathologies. Des exemples de genouillères comme dans le préambule de la revendication 1 peuvent être trouvés, par exemple, dans les documents US4494534A, US6524265B2 et US2020/197209A1.

Toutefois, les genouillères connues présentent souvent l'inconvénient de glisser le long de la jambe, de sorte que le maintien de l'articulation du genou n'est plus ou pas correctement assuré. Il existe donc un besoin d'amélioration en ce sens.

### Exposé de l'invention

Un mode de réalisation concerne une genouillère configurée pour restreindre tout ou partie des mouvements d'une articulation d'un genou lorsque la genouillère est positionnée sur un membre inférieur qui comprend une cuisse, une articulation de genou, une jambe, une articulation de cheville et un pied, l'articulation de cheville présentant deux malléoles et un tendon d'Achille, le pied présentant un cou-de-pied, la genouillère comprenant une portion de support configurée pour coopérer avec les malléoles et/ou le cou-de-pied de manière à limiter un mouvement de translation de la genouillère le long du membre inférieur vers la cheville.

On comprend que la portion de support peut être configurée pour coopérer uniquement avec les malléoles ou uniquement avec le cou-de-pied ou alors coopérer avec les malléoles et le cou-de-pied simultanément.

Une telle genouillère permet de restreindre la flexion du genou de l'utilisateur. Dans certains configurations, cette restriction peut aller jusqu'à maintenir le membre inférieur de l'utilisateur dans une position étendue et non fléchie. En toute circonstances, la genouillère restreint tout ou partie des mouvements du genou de l'utilisateur tout au long de l'utilisation de ladite genouillère.

En particulier, l'utilisation d'une portion de support entrave le mouvement de translation de la genouillère le long du membre inférieur, vers le pied, ce qui a pour effet de maintenir la genouillère en place tout au long de son utilisation. On comprend que la genouillère conserve plus facilement sa position privilégiée autour du membre inférieur et donc autour du genou. L'efficacité de la restriction de mouvement du genou de la genouillère est donc augmentée.

Par ailleurs, puisque la genouillère est maintenue en place de façon plus sûre, cette dernière frotte moins contre la peau de l'utilisateur. Par conséquent, le confort pour l'utilisateur et la durée de vie de la genouillère sont améliorés.

Dans certains modes de réalisations, la portion de support peut présenter une première portion de protection configurée pour protéger les malléoles et/ou le cou-de-pied et/ou une deuxième portion de protection configurée pour protéger le tendon d'Achille.

On comprend que la première portion de protection peut protéger uniquement les malléoles ou uniquement le cou-de-pied ou alors protéger les malléoles et le cou-de-pied simultanément.

Ceci permet d'amoindrir les inconforts que pourraient subir l'utilisateur dû au frottement de la portion de support sur les différentes parties de la cheville.

Dans certains modes de réalisations, la genouillère peut s'étendre selon une direction longitudinale et comprend une portion de genouillère adjacente à la portion de support selon la direction longitudinale.

Dans le présent exposé, la direction longitudinale correspond à la direction du membre inférieur lorsqu'un utilisateur utilise la genouillère, par exemple jambe tendue.

La portion de genouillère facilite la coopération entre les différents éléments de la genouillère. En particulier, la portion de genouillère donne un support sur lequel les autres éléments de la genouillère peuvent se rattacher.

Dans certains modes de réalisations, la genouillère peut comprendre une première paire d'armatures s'étendant longitudinalement sur tout ou partie de la portion de genouillère.

La première paire d'armatures améliore le maintien du membre inférieur dans une position étendue. En particulier, elle confère une certaine rigidité à la genouillère ce qui rend la genouillère plus apte à encaisser des chocs ou des contraintes en conservant un maintien satisfaisant du genou de l'utilisateur.

Dans certains modes de réalisations, la portion de support peut comprendre au moins une portion de raccordement fixée sur la portion de genouillère.

La portion de raccordement assure une bonne liaison entre la portion de genouillère et la portion de support. La cohésion de la genouillère est donc augmentée, ce qui renforce également sa stabilité, son maintien du genou et sa durabilité.

Dans certains modes de réalisations, la portion de raccordement peut être fixée de façon amovible sur la portion de genouillère.

Dans cette configuration, la mise en place de la genouillère autour du membre inférieur de l'utilisateur est facilitée. En particulier, la genouillère peut être équipée sur un utilisateur en deux temps, en posant en premier lieu la portion de genouillère puis en serrant en second lieu la portion de support.

Dans certains modes de réalisations, la portion de support peut être fixée à la portion de genouillère par une deuxième paire d'armatures longitudinales, chaque armature étant configurée pour s'étendre longitudinalement entre une malléole et le tendon d'Achille, et au moins une portion de la première paire d'armatures s'étend longitudinalement entre les armatures de la deuxième paire d'armatures.

Dans cette configuration, la deuxième paire d'armatures n'est pas configurée pour se superposer au tendon d'Achille, ce qui augmente le confort de l'utilisateur. Par ailleurs, la présence de la deuxième paire d'armatures de part et d'autre du tendon d'Achille permet d'en améliorer sa protection contre d'éventuels chocs extérieurs.

Dans certains modes de réalisations, la portion de raccordement peut être formée par une portion de la portion de genouillère s'étendant selon la direction longitudinale.

Dans cette configuration, la portion de genouillère et la portion de support peuvent être solidarisés ensemble par l'intermédiaire de la portion de raccordement.

Dans certains modes de réalisations, la première paire d'armatures peut s'étendre sur la portion de raccordement, et les armatures de la première paire d'armatures sont espacées l'une de l'autre d'une distance comprise entre 20 et 40 mm, selon une direction transversale, perpendiculaire à la direction longitudinale.

Dans cette configuration, la première paire d'armatures s'étendant sur la portion de raccordement n'est pas configurée pour se superposer au tendon d'Achille, ce qui augmente le confort de l'utilisateur. Par ailleurs, la présence de la première paire d'armatures de part et d'autre du tendon d'Achille permet d'en améliorer sa protection contre d'éventuels chocs extérieurs.

Dans certains modes de réalisations, la portion de support peut être configurée pour être attachée autour de la cheville par un moyen de fermeture primaire de type auto agrippant, et un moyen de fermeture secondaire.

Ainsi, le serrage de la portion de support autour de la cheville est amélioré.

Dans certains modes de réalisations, la genouillère peut comprendre un moyen de fermeture secondaire.

Ainsi, le serrage de la portion de support contre la cheville de l'utilisateur est amélioré. Cela améliore également la stabilité globale de la genouillère.

Dans certains modes de réalisations, le moyen de fermeture secondaire peut être une sangle munie d'une fixation auto-agrippante.

Dans certains modes de réalisations, le moyen de fermeture secondaire peut être un bandeau élastique.

Dans certains modes de réalisations, le moyen de fermeture secondaire peut être configuré pour se positionner au-dessus des malléoles lorsque l'utilisateur porte la genouillère.

Cette configuration améliore le confort de l'utilisateur tout en augmentant la stabilité de la genouillère. En effet, en se reposant sur les malléoles, le moyen de fermeture secondaire est moins susceptible de coulisser selon la direction longitudinale.

Dans certains modes de réalisations, la deuxième paire d'armatures peut être formée par un prolongement de la première paire d'armatures.

Cette configuration simplifie la fabrication de la genouillère, car dans ce cas, uniquement une paire d'armature est nécessaire à la fabrication de la genouillère.

Dans certains modes de réalisations, les première et deuxième paires d'armatures peuvent être en métal.

Dans certains modes de réalisations, les première et deuxième paires d'armatures peuvent être en plastique (ou matériau polymérique).

Dans certains modes de réalisations, la genouillère peut être une genouillère longue d'immobilisation.

Les genouillères longues d'immobilisation sont configurées pour empêcher tous les mouvements de l'articulation du genou en maintenant le membre inférieur dans une position non fléchie (i.e. jambe tendue). De manière générale, ce type de genouillère est configurée pour être serrée autour du membre inférieur d'un utilisateur à l'aide des moyens de fixation. La genouillère est ainsi serrée autour du genou de l'utilisateur par un serrage le long du membre inférieur et assure un maintien complet du membre inférieur, empêchant la flexion du genou.

### Brève description des dessins

L'invention et ses avantages seront mieux compris à la lecture de la description détaillée faite ci-après de différents modes de réalisation de l'invention donnés à titre d'exemples non limitatifs. Cette description fait référence aux pages de figures annexées, sur lesquelles :
[Fig. 1] La figure 1 représente une genouillère selon un premier mode de réalisation.
[Fig. 2] La figure 2 représente la genouillère de la figure 1 vue de profil et équipée sur un utilisateur.
[Fig. 3] La figure 3 représente la genouillère de la figure 2 vue en coupe dans le plan III.
[Fig. 4] La figure 4 représente une genouillère selon un deuxième mode de réalisation.
[Fig. 5] La figure 5 représente la genouillère de la figure 3 équipée sur un utilisateur, vue en coupe dans le plan V.
[Fig. 6] La figure 6 représente la genouillère de la figure 3 équipée sur un utilisateur, vue en coupe dans le plan VI.

### Description des modes de réalisation

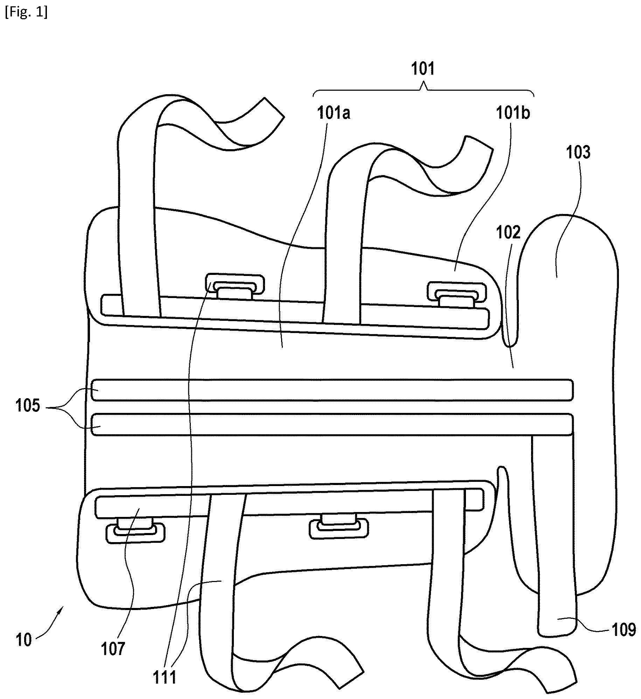

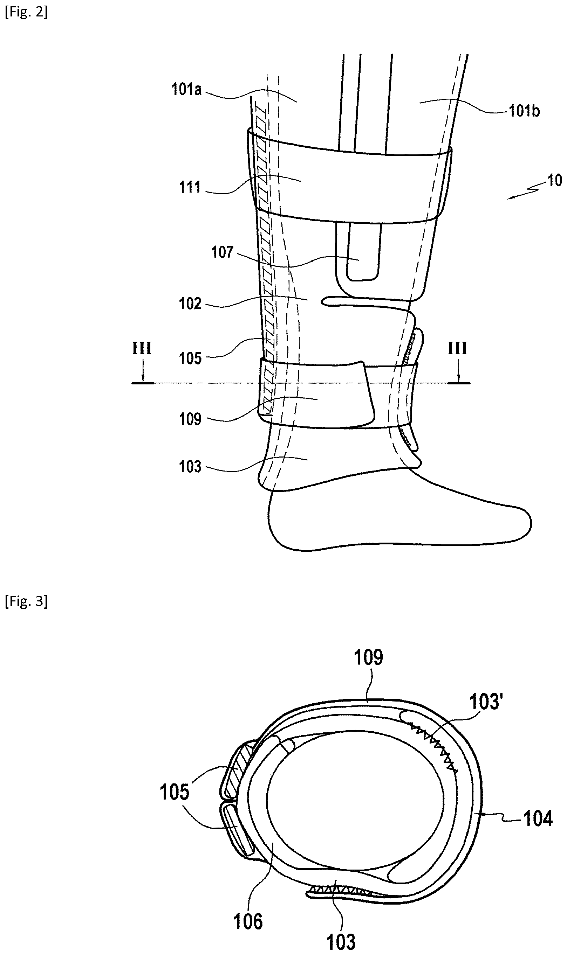

La figure 1 représente une genouillère 10 selon un premier mode de réalisation, comprenant une portion de genouillère 101, une portion de support 103, une portion de raccordement 102 configurée pour raccorder la portion de support 103 à la portion de genouillère 101 et une première paire d'armature 105 s'étendant selon une direction longitudinale X . La figure 2 représente la genouillère de la figure 1 vue de profil et équipée sur un utilisateur.

Dans le premier mode de réalisation, la paire d'armatures 105 s'étend sur la portion de genouillère 101, la portion de raccordement 102 et la portion de raccord 103. Dans cet exemple, les deux armatures de la paire d'armatures 105 sont distantes de 30 mm selon la direction perpendiculaire à la direction longitudinale. Dans cet exemple, elles sont prévues en aluminium et en forme de pavé, présentant une forme rectangulaire de 20 mm x 2,5 mm selon une section transverse à la direction longitudinale.

Dans l'exemple de ce mode de réalisation, la portion de support 103 mesure 150 mm selon la direction longitudinale X et mesure 450 mm selon une direction Y perpendiculaire à la direction longitudinale. On définit par ailleurs une direction Z perpendiculaire à la direction X et à la direction Y, correspondant à la direction de marche de l'utilisateur.

La genouillère 10 comprend un moyen de fermeture secondaire 109 attachée à une armature de la paire d'armatures 105 et à la portion de support 103, le moyen de fermeture secondaire 109 étant configuré pour serrer la portion de support 103 autour de la cheville d'un utilisateur.

Dans l'exemple du premier mode de réalisation, le moyen de fermeture secondaire 109 est de type auto-agrippant, par exemple VELCRO^{®}. Dans ce cas, la surface de la portion de support 103 sur laquelle la paire d'armatures 105 est disposée comprend un revêtement configuré pour coopérer avec le moyen de fermeture secondaire 109. Ce revêtement peut s'étendre sur une partie ou sur l'intégralité de la surface de la portion de support 103.

Comme représenté sur la figure 2, le moyen de fermeture secondaire 109 se positionne au-dessus des malléoles de l'utilisateur lorsque la portion de support 103 est serrée autour de la cheville de l'utilisateur.

La portion de genouillère 101 comprend un corps 101a et deux pans d'ajustement 101b distincts. Les pans d'ajustement 101b sont configurés pour se placer du côté avant du membre inférieur lorsque la genouillère 10 est équipée par un utilisateur.

La genouillère 10 comprend par ailleurs des parties renforcées 107, chacune disposée sur l'un des deux pan d'ajustement 101b. Une pluralité de moyens de fixation 111 sont disposés sur les parties renforcées 107, chaque moyen de fixation comprenant une sangle disposée sur l'une des parties renforcées 107 et une boucle disposée sur l'autre partie renforcée 107. La sangle et la boucle d'un moyen de fixation 111 sont configurées pour coopérer pour appliquer un serrage de la genouillère 10 autour d'un membre inférieur d'un utilisateur.

Comme représenté sur la figure 1, les moyens de fixation 111 sont arrangés en alternance sur les parties renforcées 107. En effet, sur une même partie renforcée 107, la boucle d'un moyen de fixation 111 est immédiatement précédée et immédiatement suivie d'une sangle d'un autre moyen de fixation 111.

Dans l'exemple du premier mode de réalisation, les moyens de fixation 111 sont fixés à l'aide de partie auto-agrippantes, par exemple VELCRO^{®}. Cependant, d'autres types de fixations peuvent être envisagés, comme des boucles à ouverture rapide.

Dans certains modes de réalisation, un bandeau élastique supplémentaire peut être disposé sur les moyens de fixation 111 pour améliorer la tenue du serrage de la genouillère 10 autour du membre inférieur de l'utilisateur.

Par ailleurs, dans certains modes de réalisation, la portion de raccordement 102 est amovible et peut se détacher de la portion de genouillère 101. Dans de telles configurations, la potion de raccordement 102 comprend une partie auto-aggripante et la portion de genouillère 101 comprend une partie apte à la coopération avec la partie auto-agrippante de la portion de raccordement 102. Alternativement, la portion de raccordement 102 peut être un prolongement de la portion de genouillère 101.

La figure 3 représente la genouillère de la figure 2 vue en coupe dans le plan III. La portion de support 103 comprend un moyen de fermeture primaire 103' configuré pour coopérer avec une autre partie de la portion de support 103 afin de serrer la portion de support 103 autour de la cheville d'un utilisateur.

En d'autres termes, la portion de support 103 présente un moyen de fermeture primaire 103' configuré pour serrer la portion de support 103 autour de la cheville d'un utilisateur, la portion de support 103 s'attachant sur elle-même par la coopération du moyen de fermeture primaire 103' avec une autre partie de la portion de support 103.

Dans l'exemple du premier mode de réalisation, le moyen de fermeture primaire 103' est de type auto-agrippant, par exemple VELCRO^{®}. Dans ce cas, la surface de la portion de support 103 comprend un revêtement configuré pour coopérer avec le moyen de fermeture primaire 103'. Ce revêtement peut s'étendre sur une partie ou sur l'intégralité de la surface de la portion de support 103.

Dans le présent exemple, le moyen de fermeture secondaire 109 décrit précédemment est configuré pour se refermer par-dessus la portion de support 103 et donc par-dessus le moyen de fermeture primaire 103'.

Par ailleurs, comme cela est illustré sur la figure 3, l'enroulement de la portion de support 103 autour de la cheville est tel que dans une zone située au niveau du cou-de-pied, la portion de support 103 forme un empilement selon son épaisseur qui définit une première portion de protection 104.

La portion de support 103 comprend par ailleurs une deuxième portion de protection 106 configurée pour faire face au tendon d'Achille lorsque la genouillère 10 est équipée par un utilisateur. De cette façon, la deuxième portion de protection 106 protège le tendon d'Achille.

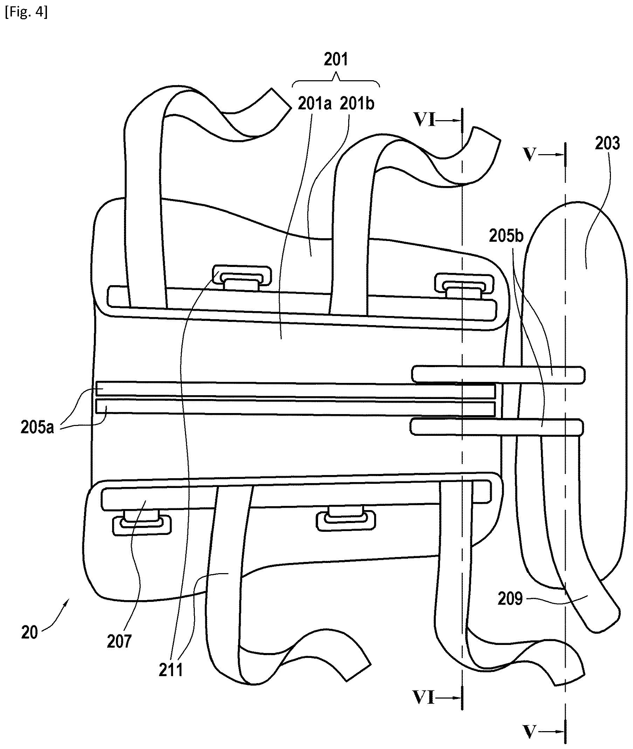

La figure 4 représente une genouillère 20 selon un deuxième mode de réalisation. La genouillère 20 comprend une portion de genouillère 201, 201a, 201b, une portion de support 203 présentant un moyen de fermeture primaire 203', des moyens de fixation 211 fixés sur des parties renforcées 207 et un moyen de fermeture secondaire 209. Ces éléments sont en tous points comparables aux éléments homonymes du premier mode de réalisation. Par ailleurs, la genouillère 20 peut comprendre une portion de raccordement non représentée, cette portion de raccordement étant analogue à la portion de raccordement 102 du premier mode de réalisation.

Dans le deuxième mode de réalisation, la portion de genouillère 201 et la portion de support 203 sont deux parties distinctes de la genouillère 20. Il est entendu que le mot « distinctes » désigne deux pièces d'un même ensemble identifiables séparément l'une de l'autre ou deux éléments différents. Dans l'exemple du deuxième mode de réalisation, la portion de support 203 et la portion de genouillère 201 sont distantes de 0 à 100 mm.

Par ailleurs, la genouillère 20 comprend une première paire d'armatures 205a et une deuxième paire d'armatures 205b. La première paire d'armatures 205a s'étend selon la direction longitudinale sur la portion de genouillère 201et la deuxième paire d'armatures 205b s'étend selon la direction longitudinale sur la portion de genouillère 201 et sur la portion de support 203.

La deuxième paire d'armatures 205b s'étend partiellement sur la portion de support 203, de sorte que l'extrémité distale des armatures 205b dans la direction longitudinale et l'extrémité distale de la portion de support 203 selon la direction longitudinale soient par exemple distantes d'au moins 50 mm.

La première paire d'armatures 205a s'étend longitudinalement entre les armatures de la deuxième paire d'armatures 205b sur la portion de genouillère 201. Les armatures de la première paire d'armatures 205a sont par exemple espacées de 5 mm tandis que les armatures de la deuxième paire d'armatures 205b sont espacées par exemple de 50 mm.

Les armatures de la première paire d'armatures 205a et de la deuxième paire d'armatures 205b sont identiques aux armatures de la première paire d'armatures 105 du premier mode de réalisation, à l'exception de leur dimension longitudinale.

La deuxième paire d'armatures 205b est configurée pour pouvoir se fixer à la portion de genouillère 201 et à la portion de support 203. Par conséquent, la deuxième paire d'armatures 205b assure le raccordement entre la portion de genouillère 201 et la portion de support 203.

On comprend que la portion de support 203 est rattachée à la portion de genouillère 201 par l'intermédiaire de la deuxième paire d'armatures 205b. Ce rattachement peut être réversible ou permanent. En d'autres termes, la portion de support 203 peut être amovible et être détachée de la portion de genouillère 201, ou peut y être fixée de manière permanente. Par ailleurs, d'autres attaches additionnelles, réversibles ou permanentes, peuvent être prévues entre la portion de support 203 et la portion de genouillère 201, comme une portion de raccordement analogue à la portion de raccordement 102 du premier mode de réalisation.

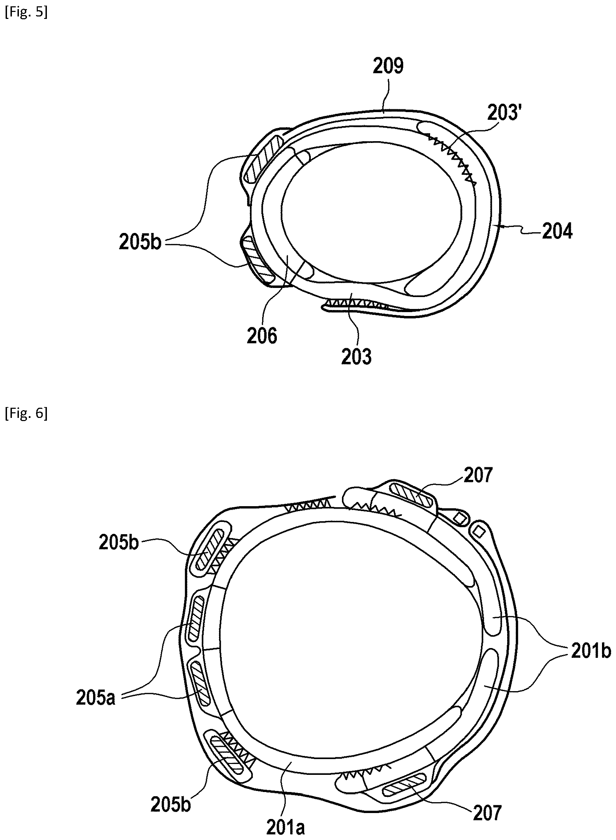

La figure 5 représente une vue en coupe dans le plan V de la genouillère 20 de la figure 4 équipée par un utilisateur. On note que la portion de support 203 du deuxième mode de réalisation peut comprendre une première portion de protection 204 et une deuxième portion de protection 206 correspondant aux portions de protections 104, 106 décrits précédemment.

La figure 6 représente une vue en coupe dans le plan VI de la genouillère 20 de la figure 4 équipée par un utilisateur. Lorsque la genouillère 20 est équipée par un utilisateur, les pans d'ajustement 201b sont configurés pour se placer au niveau du tibia de l'utilisateur. Par ailleurs, les pans d'ajustement 201b sont configurés pour ajuster la circonférence de la genouillère 20 en fonction du membre inférieur de l'utilisateur. Ainsi une genouillère 20 peut s'adapter à des circonférences de jambes diverses.

On note par ailleurs que les pans d'ajustement 101b de la portion de genouillère 101 du premier mode de réalisation peuvent aussi être configurés pour adapter la circonférence de la genouillère 10 en fonction du membre inférieur de l'utilisateur.

Bien que la présente invention ait été décrite en se référant à des exemples de réalisation spécifiques, il est évident que des modifications et des changements peuvent être effectués sur ces exemples sans sortir de la portée générale de l'invention telle que définie par les revendications. En particulier, des caractéristiques individuelles des différents modes de réalisation illustrés/mentionnés peuvent être combinées dans des modes de réalisation additionnels. Par conséquent, la description et les dessins doivent être considérés dans un sens illustratif plutôt que restrictif.

Il est également évident que toutes les caractéristiques décrites en référence à un dispositif sont transposables, seules ou en combinaison, à un procédé.

## Revendications

1. Genouillère (20) configurée pour restreindre tout ou partie des mouvements d'une articulation d'un genou lorsque la genouillère (20) est positionnée sur un membre inférieur qui comprend une cuisse, une articulation de genou, une jambe, une articulation de cheville et un pied, l'articulation de cheville présentant deux malléoles et un tendon d'Achille, le pied présentant un cou-de-pied, la genouillère (20) comprenant une portion de support (203) configurée pour coopérer avec les malléoles et/ou le cou-de-pied de manière à limiter un mouvement de translation de la genouillère (20) le long du membre inférieur vers la cheville, dans laquelle la genouillère s'étend selon une direction longitudinale et comprend une portion de genouillère (201) adjacente à la portion de support (203) selon la direction longitudinale, la genouillère comprend une première paire d'armature (205a) s'étendant longitudinalement sur tout ou partie de la portion de genouillère (203) et dans laquelle la portion de support est fixée à la portion de genouillère (201) par une deuxième paire d'armatures longitudinales (205b), **caractérisé en ce que** au moins une portion de la première paire d'armatures (205a) s'étend longitudinalement entre les armatures de la deuxième paire d'armatures (205b).

2. Genouillère selon la revendication 1, dans laquelle la portion de support (203) comprend au moins une portion de raccordement fixée sur la portion de genouillère (201).

3. Genouillère (10) configurée pour restreindre tout ou partie des mouvements d'une articulation d'un genou lorsque la genouillère (10) est positionnée sur un membre inférieur qui comprend une cuisse, une articulation de genou, une jambe, une articulation de cheville et un pied, l'articulation de cheville présentant deux malléoles et un tendon d'Achille, le pied présentant un cou-de-pied, la genouillère (10) comprenant une portion de support (103) configurée pour coopérer avec les malléoles et/ou le cou-de-pied de manière à limiter un mouvement de translation de la genouillère (10) le long du membre inférieur vers la cheville, dans laquelle la genouillère s'étend selon une direction longitudinale et comprend une portion de genouillère (101) adjacente à la portion de support (103) selon la direction longitudinale, la genouillère comprend une première paire d'armature (105) s'étendant longitudinalement sur tout ou partie de la portion de genouillère (103), la portion de support (103) comprend au moins une portion de raccordement (102) fixée sur la portion de genouillère (101), dans laquelle la portion de raccordement (102) est formée par une portion de la portion de genouillère (101) s'étendant selon la direction longitudinale, et dans laquelle la première paire d'armatures (105) s'étend sur la portion de raccordement, et les armatures de la première paire d'armatures (102) sont espacées l'une de l'autre d'une distance comprise entre 20 et 40 mm, selon une direction transversale, perpendiculaire à la direction longitudinale.

4. Genouillère (10,20) selon l'une des revendications 1 à 3, dans laquelle la portion de support (103,203) présente une première portion de protection (204) configurée pour protéger les malléoles et/ou le cou-de-pied et/ou une deuxième portion de protection (206) configurée pour protéger le tendon d'Achille.

5. Genouillère (10,20) selon l'une des revendications 2 à 4, dans laquelle la portion de raccordement (102) est fixée de façon amovible sur la portion de genouillère (101,201).

6. Genouillère (10,20) selon l'une quelconque des revendications 1 à 5, dans laquelle la portion de support (103,203) est configurée pour être attachée autour de la cheville par un moyen de fermeture primaire (103') de type auto agrippant, et un moyen de fermeture secondaire (109).

## Patentansprüche

1. Knieorthese (20), die dazu ausgestaltet ist, Bewegungen eines Gelenks eines Knies ganz oder teilweise zu beschränken, während die Knieorthese (20) an einer unteren Gliedmaße positioniert ist, die einen Schenkel, ein Kniegelenk, ein Bein, ein Sprunggelenk und einen Fuß umfasst, wobei das Sprunggelenk zwei Knöchel und eine Achillessehne aufweist, wobei der Fuß einen Rist aufweist, wobei die Knieorthese (20) einen Stützabschnitt (203) umfasst, der dazu ausgestaltet ist, mit den Knöcheln und/oder dem Rist derart zusammenzuwirken, dass eine Translationsbewegung der Knieorthese (20) entlang der unteren Gliedmaße in Richtung des Sprunggelenks begrenzt wird,
wobei sich die Knieorthese gemäß einer Längsrichtung erstreckt und einen Knieorthesenabschnitt (201) umfasst, der an den Stützabschnitt (203) gemäß der Längsrichtung angrenzt,
wobei die Knieorthese ein erstes Paar Streben (205a) umfasst, das sich längs ganz oder teilweise über den Knieorthesenabschnitt (203) erstreckt, und
wobei der Stützabschnitt an dem Knieorthesenabschnitt (201) durch ein zweites Paar Längsstreben (205b) befestigt ist, **dadurch gekennzeichnet, dass** sich mindestens ein Abschnitt des ersten Paars Streben (205a) längs zwischen den Streben des zweiten Paars Streben (205b) erstreckt.

2. Knieorthese nach Anspruch 1, wobei der Stützabschnitt (203) mindestens einen Verbindungsabschnitt umfasst, der an dem Knieorthesenabschnitt (201) befestigt ist.

3. Knieorthese (10), die die dazu ausgestaltet ist, Bewegungen eines Gelenks eines Knies ganz oder teilweise zu beschränken, während die Knieorthese (10) an einer unteren Gliedmaße positioniert ist, die einen Schenkel, ein Kniegelenk, ein Bein, ein Sprunggelenk und einen Fuß umfasst, wobei das Sprunggelenk zwei Knöchel und eine Achillessehne aufweist, wobei der Fuß einen Rist aufweist, wobei die Knieorthese (10) einen Stützabschnitt (103) umfasst, der dazu ausgestaltet ist, mit den Knöcheln und/oder dem Rist derart zusammenzuwirken, dass eine Translationsbewegung der Knieorthese (10) entlang der unteren Gliedmaße in Richtung des Sprunggelenks begrenzt wird,
wobei sich die Knieorthese gemäß einer Längsrichtung erstreckt und einen Knieorthesenabschnitt (101) umfasst, der an den Stützabschnitt (103) gemäß der Längsrichtung angrenzt,
wobei die Knieorthese ein erstes Paar Streben (105) umfasst, das sich längs ganz oder teilweise über den Knieorthesenabschnitt (103) erstreckt,
wobei der Stützabschnitt (103) mindestens einen Verbindungsabschnitt (102) umfasst, der an dem Knieorthesenabschnitt (101) befestigt ist,
wobei der Verbindungsabschnitt (102) durch einen Abschnitt des Knieorthesenabschnitts (101) gebildet ist, der sich gemäß der Längsrichtung erstreckt, und
wobei sich das erste Paar Streben (105) über den Verbindungsabschnitt erstreckt und die Streben des ersten Paars Streben (102) um einen Abstand zwischen 20 und 40 mm gemäß einer Querrichtung senkrecht zu der Längsrichtung voneinander beabstandet sind.

4. Knieorthese (10, 20) nach einem der Ansprüche 1 bis 3, wobei der Stützabschnitt (103, 203) einen ersten Schutzabschnitt (204), der dazu ausgestaltet ist, die Knöchel und/oder den Rist zu schützen, und/oder einen zweiten Schutzabschnitt (206), der dazu ausgestaltet ist, die Achillessehne zu schützen, aufweist.

5. Knieorthese (10, 20) nach einem der Ansprüche 2 bis 4, wobei der Verbindungsabschnitt (102) unbeweglich an dem Knieorthesenabschnitt (101, 201) befestigt ist.

6. Knieorthese (10, 20) nach einem der Ansprüche 1 bis 5, wobei der Stützabschnitt (103, 203) dazu ausgestaltet ist, um das Sprunggelenk herum durch ein primäres Verschlussmittel (103') vom Typ Klettverschluss und ein sekundäres Verschlussmittel (109) angebracht zu werden.

## Claims

1. A knee pad (20) configured to restrict all or part of the movements of a knee joint when the knee pad (20) is positioned on a lower limb which comprises a thigh, a knee joint, a leg, an ankle joint and a foot, the ankle joint having two malleoli and an Achilles tendon, the foot having an instep, the knee pad (20) comprising a support portion (203) configured to cooperate with the malleoli and/or the instep so as to limit a translational movement of the knee pad (20) along the lower limb towards the ankle,
wherein the knee pad extends along a longitudinal direction and comprises a knee portion (201) adjacent to the support portion (203) along the longitudinal direction,
the knee pad comprises a first pair of reinforcements (205a) extending longitudinally over all or part of the knee pad portion (203), and
wherein the support portion is fixed to the knee pad portion (201) by a second pair of longitudinal reinforcements (205b), **characterized in that** at least one portion of the first pair of reinforcements (205a) extends longitudinally between the reinforcements of the second pair of reinforcements (205b).

2. The Knee pad according to claim 1, wherein the support portion (203) comprises at least one connection portion fixed on the knee pad portion (201).

3. The Knee pad (10) configured to restrict all or part of the movements of a knee joint when the knee pad (10) is positioned on a lower limb which comprises a thigh, a knee joint, a leg, an ankle joint and a foot, the ankle joint having two malleoli and an Achilles tendon, the foot having an instep, the knee pad (10) comprising a support portion (103) configured to cooperate with the malleoli and/or the instep so as to limit a translational movement of the knee pad (10) along the lower limb towards the ankle,
wherein the knee pad extends along a longitudinal direction and comprises a knee pad portion (101) adjacent to the support portion (103) along the longitudinal direction,
the knee pad comprises a first pair of reinforcements (105) extending longitudinally over all or part of the knee pad portion (103),
the support portion (103) comprises at least one connection portion (102) fixed on the knee pad portion (101),
wherein the connection portion (102) is formed by a portion of the knee pad portion (101) extending along the longitudinal direction, and
wherein the first pair of reinforcements (105) extends over the connection portion, and the reinforcements of the first pair of reinforcements (102) are spaced from each other by a distance comprised between 20 and 40 mm, along a transverse direction, perpendicular to the longitudinal direction.

4. The Knee pad (10, 20) according to any of claims 1 to 3, wherein the support portion (103, 203) has a first protective portion (204) configured to protect the malleoli and/or the instep and/or a second protective portion (206) configured to protect the Achilles tendon.

5. The Knee pad (10, 20) according to any of claims 2 to 4, wherein the connection portion (102) is removably fixed on the knee pad portion (101, 201).

6. The Knee pad (10, 20) according to any one of claims 1 to 5, wherein the support portion (103, 203) is configured to be attached around the ankle by a primary closing means (103') of the hook-and-loop type, and a secondary closing means (109).
